# EUROPEAN PATENT APPLICATION

(11) **EP 4 538 982 A1**
(43) Date of publication of application: **16.04.2025**
(21) Application number: 23202665.8
(22) Date of filing: 10.10.2023
(51) Int. Cl.: G06T 19/20, G06T 7/00, G16H 30/40

(54) **COMPUTER IMPLEMENTED METHOD FOR ANALYSING A HEART VALVE, COMPUTER PROGRAM AND SYSTEM**

(71) Applicant: Koninklijke Philips N.V., 5656 AG Eindhoven (NL)
(72) Inventor: BLITZ, Alexandra, Eindhoven (NL); BLANKENHAGEN, Michael, Eindhoven (NL); SCHRECKENBERG, Marcus, 5656AG Eindhoven (NL)
(74) Representative: Philips Intellectual Property & Standards

(57) **Abstract**

The invention is directed to a method for analysing a heart valve (52) of a subject, comprising the steps of: (a) providing a 3D or 4D volume data set (32) of a heart valve (52; (b) providing a valve model (2, 3) of the heart valve (52), wherein the valve model (2, 3) is adjusted to the morphology of the heart valve (52) and comprises a closed curve (9, 12) representing the annulus of the valve, wherein the area within the closed curve (9, 12) comprises at least one 2D section (16), each 2D section (16) corresponding to a morphological or physiological feature of the heart valve (52); and (c) projecting the valve model (2, 3) into the 3D or 4D volume data set (32) along a projection direction (36) to define a volume-of-interest (42), wherein the volume-of-interest (42) includes at least one 3D region (26), each 3D region (26) being defined by the projection of a corresponding 2D section of the at least one 2D section (16); (d) labelling each of the voxels in the at least one 3D region (26) with a label indicative of the morphological or physiological feature of the corresponding 2D section (16). Preferably, a visualisation of a cut plane through the 2D or 3D volume data set is provided, in which voxels in at least one 3D region (26) of the volume-of-interest (42) are represented in a colour depending on their label.

## Description

### FIELD OF THE INVENTION

The present invention relates to a computer-implemented method for analysing a heart valve of a subject, a computer program and a system for analysing a heart valve.

### BACKGROUND OF THE INVENTION

Three-dimensional (3D) echocardiography is a common method to visualize the human heart and in particular the heart valves. When 3D ultrasound images are acquired of the moving heart over a period of time, resulting in a video clip, also termed 3D echo clip, this is termed four-dimensional (4D) echocardiography. 3D or 4D echocardiography is commonly used to plan and perform minimally invasive heart surgery, such as trans-catheter valve procedures. For example, valve repair through leaflet clipping is becoming a common method to reduce regurgitant blood flow and improve the patient's quality of life significantly. However, such procedures need to be very carefully and precisely planned. The interventional cardiologists need to know which leaflets they have to clip during the procedure. Therefore, a good planning tool is required, as well as a good method of visualising the heart valves during the interventional procedure.

It is known to provide volume-rendered images of the heart and parts thereof, such as a heart valve. However, many surgeons and echocardiographers still prefer to rely on two-dimensional (2D) planes through the 3D ultrasound images. However, due to the complexity of the anatomical structures of the heart valve, it may be extremely difficult to maintain orientation in relation to the valve anatomy on such 2D planes.

US 2005187461A discloses a computerized method of facilitating cardiac intervention, comprising inputting patient data, creating a computerized interactive model of a heart based on the patient data, simulating at least one proposed cardiac intervention treatment by adding or deleting features to the model, and determining the effects of the proposed cardiac simulation upon the entire model. Simulations may be repeated to allow the user to determine an optimal cardiac intervention. Additionally, a template may be created from the model to use as a guide during the cardiac intervention.

US 2020082531 A1 shows a device for dynamically assessing a moving object from a sequence of consecutive volumetric image frames of such object, which images are timely separated by a certain time interval, by: identifying in at least one image of the sequence the object of interest; segmenting the object to identify object contour; propagating the object contour as identified to other images of the sequence; and performing dynamic analysis of the object based on the object contour as propagated.

The White Paper "Quantifying Heart Valves: From Diagnostic to Personalized Valve Repair" by Tommaso Mansi et al., 04 2016, describes a method of valve modelling and editing. Therein it is proposed to provide a combination of editing views, such as parallel cuts or rotational cuts and smart mesh editing.

However, assessing the topology of a heart valve remains cumbersome, and preparing surgical procedures is therefore still difficult.

### OBJECT OF THE INVENTION

Therefore, it is an object of the present invention to provide a method and device which may provide a better and more intuitive way to assess anatomical structures, in particular the structures like the heart valve, which are complex and hard to visualise.

### SUMMARY OF THE INVENTION

The present invention solves this problem with a computer-implemented method including the features of claim 1, with a computer program including the features of claim 14 and a system including the features of claim 15.

According to one aspect, the present invention provides a computer-implemented method for analysing a heart valve of a subject, the method comprising the steps of:
(a) providing a 3D or 4D volume data set of a heart valve of a subject;
(b) providing a valve model of the heart valve, wherein the valve model is adjusted to the morphology of the heart valve, and
   wherein the valve model comprises a closed curve representing the annulus of the valve, wherein the area within the closed curve comprises at least one 2D section, each 2D section corresponding to a morphological or physiological feature of the heart valve; and
(c) projecting the valve model into the 3D or 4D volume data set along a projection direction to define a volume-of-interest, wherein the volume-of-interest includes at least one 3D region, each 3D region being defined by the projection of a corresponding 2D section of the at least one 2D section;
(d) labelling each of the voxels in the at least one 3D region with a label indicative of the morphological or physiological feature of the corresponding 2D section.

The invention is based on the idea of transforming anatomical knowledge about the heart valve, which is manifested in a valve model, into a labelling of the voxels within the 3D or 4D volume data set, which may greatly facilitate navigating through the 3D or 4D volume data set. In particular, different regions (2D sections) within the heart valve, such as the different leaflets, can be determined in a simplified representation (the valve model) of the heart valve, but the information contained therein can be projected over the volume data set. The user can then navigate through the structures in the 3D or 4D image (3D or 4D volume data set) without having to switch between image information and model information, because the labelling of the voxels may be used to represent the 3D regions corresponding to different morphological or physiological features of the heart valve differently, for example in different colours.

The method of the invention is performed on a computer, wherein the computer may be connected to an ultrasound acquisition system, it may for example be its control computer. The computer may also be a stand-alone computer, on which data analysis is carried out. Thus, the computer may be any PC, workstation, cloud computer, tablet, laptop or mobile device.

The method of the invention is based on a valve model, which is preferably a highly simplified model of the valve on a 2D or 3D surface which is enclosed within a closed curve representing the annulus, Preferably, it is two-dimensional (2D) in the sense that any extension of the annulus or leaflets into a direction perpendicular to the main plane of the annulus is not represented in the valve model, but projected into the surface within the closed curve. This surface, however, may be three-dimensional (3D), in order to best fit the heart valve. For example, the valve model may have the shape of a potato chip. In this case, it is termed a 3D valve model herein. In another embodiment the valve model may be a 2D model in the sense that the surface enclosed by the closed curve is on a plane, in particular a plane corresponding to the main plane of the annulus. An example of such a 2D model is the topology-based 2D model explained herein below. The "main plane of the annulus" is a plane which may describe the orientation of the heart valve, e.g. a plane that best fits the annulus or the corresponding 3D valve model, even though the annulus is not exactly lying in this plane due to its three-dimensional shape.

The valve model comprises a closed curve representing the annulus. The closed curve preferably may have any 3D shape, as long as it is closed. Such a 3D closed curve is also referred to herein as 3D ring. A 3D ring may for example have the shape of the outer circumference of a potato chip. The closed curve of the valve model may be derived from a segmentation of the annulus. In other embodiments, or in further simplified models which may be derived from the valve model, the closed curve may have a standardized shape, for example a circle, ellipse, polygon, or polygon with rounded edges.

The valve model is preferably a simplified visualisation of the main elements of the heart valve, in particular the annulus and the leaflets. The area within the closed curve comprises at least one 2D section, wherein the at least 2D section represents a morphological or physiological feature of the heart valve. A morphological feature may be an anatomical structure in the heart valve, for example a leaflet or a papillary muscle. In particular, at least one 2D section may represent a leaflet of the heart valve. Thus, the valve model may comprise information on the position, area and/or shape of the leaflets. For example, the leaflets may be characterised by commissure points defined on the closed curve, wherein each commissure point defines one end of a commissure line between two leaflets of the heart valve. The other end of the commissure line may be a central point of the valve model. A commissure line may be a division between two 2D sections. A physiological feature may be a blood flow phenomenon, such as regurgitant flow.

The valve model is derived from the 3D or 4D volume data set, preferably automatically or semi-automatically. In a semi-automatic embodiment, an initial valve model may be automatically created. This may be performed by first segmenting the heart valve from the 3D or 4D volume data set, and then fitting a 3D valve model to the segmented heart valve, in particular to the annulus. The user may adjust the 3D valve model when it is overlaid on a visualisation of the volume data set or the segmented heart valve, for example within a volume rendering. The 3D valve model may further be converted into, in particular mapped onto, a standardised topology-based 2D model, and the user may be given the opportunity to adjust the topology-based 2D model to the visualisation of the heart valve, for example by adjusting the position of commissure points. This is explained below in more detail. Preferably, the topology-based 2D model is a static, symbolic representation of the heart valve, in that it does not change over a heart cycle. Thus, even if the 3D valve model of the heart valve moves over a heartbeat, the topology-based 2D model will remain static.

The 3D or 4D volume data set may be or have been obtained by a medical imaging modality, preferably by ultrasound, for example by 3D or 4D echocardiography, in particular by transoesophageal 4D echocardiography. Other possible imaging modalities are magnetic resonance imaging (MRI), X-ray imaging, Positron Emission Spectroscopy (PET), or computed tomography (CT). A 3D volume data set comprises a 3D matrix of voxels, each voxel comprising a greyscale value representing image information. In a 4D volume data set, the fourth dimension is time. In other words, a 4D volume data set may be a video clip containing a time series of frames (i.e., 3D volume data sets) covering for example, one or several heartbeat cycles. Thus, the 4D volume data set may be acquired as a sequence of 3D images obtained from a human or animal subject spanning a time period with a frame rate of for example of 5-100, preferably 20-60 images per second, so as to allow a smooth representation of the dynamically moving heart valve. The time period is usually at least one cycle of a cyclical movement of the heart, e.g., at least one heartbeat. The subject may be a human or animal, in particular a patient. The heart valve may be any of the four heart valves, for example the mitral valve or the tricuspid valve. The 3D or 4D volume data set may be provided in DICOM (Digital Imaging and Communications in Medicine) format, or any other suitable image format. The 3D or 4D volume data set, also referred to as volume data set, image data set or image herein, includes image data comprising morphological information on an anatomical structure, namely the heart or part of the heart. The morphological information of the heart valve in particular comprises anatomical features, such as the shape, structure, size etc., of the constituent parts of the heart valve like the leaflets and annulus. Thus, a morphological feature of a heart valve may for example be a leaflet, part of a leaflet or any other part thereof. The physiology of the heart valve, by contrast, deals primarily with function. Thus, a physiological feature of the heart valve may for example be an area of irregular blood flow within the heart, for example the position of a regurgitant leakage, and more generally the location and magnitude of a flow phenomenon.

The valve model may be derived from the 3D or 4D volume data set for example by the following method: First, the 3D or 4D volume data set is acquired of the moving heart as described above, wherein the data set comprises image data of the heart valve. In a next step, the heart valve is segmented. Thereby, the anatomical structures of the heart valve are separated from blood or other tissue, so that preferably only voxels which are part of the heart valve or the immediate surroundings remain. Segmentation methods are generally known in the art. For example, the segmentation may be carried out as shown in the Dröge, Hannah, et al. "Mitral Valve Segmentation Using Robust Nonnegative Matrix Factorization". Journal of Imaging, 2021, Vol. 7, No. 10, page 213, which is incorporated herein by reference.

From the segmented heart valve, the valve model may be derived. In preferred embodiments, the valve model is or is based on a 3D valve model of the heart valve. The 3D valve model is preferably dynamic, i.e., it follows the movement of the anatomical structure, namely the heart valve, across the time period. The dynamic 3D valve model can be used to visualize the essential constituent components of the heart valve, in particular the annulus and possibly the leaflets. In a preferred embodiment, the 3D valve model comprises a 3D ring representing the annulus, which is positioned and oriented within the 3D or 4D volume data set at the position and orientation of the annulus of the heart valve. Such 3D ring may be obtained by fitting a 3D ring, in other words a three-dimensional closed curve which may have any shape in three dimensions, to the segmented heart valve. It may also be obtained by manually or automatically or semi-automatically identifying certain landmarks of the heart valve on the 3D or 4D volume data, or on the segmented heart valve, and then fitting a 3D ring to the landmarks. Alternatively, the 3D valve model may be a shape/surface model of the heart valve. The surface 3D valve model may be a simplification of the heart valve, for example a triangulated surface covering the annulus. The 3D valve model may comprise a number of points spanning a three-dimensional closed curve, or alternatively a surface, for each frame of the 3D or 4D volume data set. It may also be a mathematical model, for example a parametrized model, such as a closed ring, surface or volume spanned by spline curves. The 3D valve model may be automatically fitted to the segmented heart valve. In some embodiments, the user may be given the opportunity to adjust the 3D valve model on a visualisation of the heart valve.

Within the closed curve of the valve model, which may be or may correspond to the 3D valve model, 2D sections may be defined. For example, anatomical features of the heart valve may be represented in the valve model by the commissure points, which are positioned on the closed curve. In addition, lines separating the leaflets from one another may be represented on the valve model. Such commissure lines may start from a commissure point and extend towards a central point of the valve model, or to another point within the closed curve, which corresponds to an end point of the leaflet. The central point may be the centre of gravity of the closed curve/3D ring of the 3D valve model representing the annulus. The sections separated by the commissure lines may thus represent 2D sections corresponding to morphological features, namely leaflets. Other morphological features may also be represented as 2D sections, e.g. areas of leaflet damage. In addition, physiological features such as certain areas or 2D sections, in which regurgitant blood flow occurs at least during a part of the heart cycle, may be represented on the valve model. Preferably, the valve model is automatically created. In some embodiments, the user may adjust the valve model such that it fits best to the 3D or 4D volume data set, as visualised in a visualisation environment.

In an embodiment, the valve model is based on or is correlated to a topology-based 2D model of the heart valve. The topology-based 2D model may be derived from the 3D valve model. The topology-based 2D model is essentially a further simplification of the 3D valve model, in that the 2D model does not extend into the third dimension, in particular not in a dimension perpendicular to the main plane of the annulus. Thus, the topology-based 2D model may be derived by projecting or mapping the 3D valve model onto a plane. When mapping the 3D valve model onto a plane to obtain the topology-based 2D model, the proportions of the annulus of the 3D valve model may be preserved. In other embodiments, the projection of the 3D valve model onto a 2D surface may be distorted to obtain the topology-based 2D model. In another embodiment, the topology-based 2D model is created based on a developable surface of the segmented heart valve or the 3D valve model. A developable surface is a surface that can be flattened onto a plane without distortion, i.e., by "folding", "bending", etc. In this case, the topology-based 2D model may be the developable surface enclosed by the 3D ring of the 3D valve model, flattened onto a 2D plane. The thus obtained "flattened" 2D closed curve may be further simplified by transforming it to a standardised shape, such as a circle, or an ellipse.

Preferably, the valve model, in particular the 3D valve model, is tracked over at least one heartbeat in a 4D volume data set. In other words, the valve model can be visualised in any of the frames, and preferably adapted by a user to best fit the segmented heart valve, or a 3D or 4D visualisation of the heart valve or the segmented heart valve. The mapping of the 3D valve model to the topology-based 2D model is also tracked over the heartbeats, so that the models can be visualised at any time point during the heart cycle. However, the topology-based 2D model is preferably static, in that it does not change over a heartbeat.

The topology-based 2D model is preferably a standardised simplification of an actual segmentation of the heart valve. Thereby, a 3D representation of the heart valve, for example the segmented heart valve, is transferred to a 2D visualisation of the heart valve. Such standardised representation of a heart valve is highly useful, since it has been found that heart valves may differ considerably in their morphology, to an extent that even the number of leaflets may differ between patients, in particular some may have two leaflets, other may have three leaflets, and a standardised representation thereof may considerably help in diagnosis.

Although the topology-based 2D model is useful in visualising the heart valve, the physician may still need to refer to the original 3D or 4D volume data set in order to plan heart surgery, or in order to direct surgical instruments during a heart surgery. A visualisation of the 3D or 4D volume data set is thereby used, wherein the visualisation may for example be a 3D rendering, for example a volume rendering, or alternatively or additionally a 2D cut plane through the 3D or 4D volume data set. Such cut plane may be generated by multi-planar reconstruction (MPR), wherein the pixels on a plane which is oriented at an oblique angle through the original 3D image matrix, are calculated e.g. by interpolation from the nearest voxels of the original volume data set. Thereby, the cut plane may be oriented in any user-selected orientation through the 3D or 4D image data set. Such cut planes are often used by physicians when assessing 3D or 4D volume data sets of heart valves, wherein the physician will shift and tilt the cut plane at various angles to view different portions of the 3D or 4D volume dataset. The 2D cut plane may usefully be oriented along the axis of a heart chamber, for example a ventricle (LaX planes). However, the 2D cut plane may also be oriented orthogonal to this axis (SaX planes). It has been found that image analysis and orientation within the data is facilitated by the visualisation method of the invention in any kind of cut planes.

The invention has recognised that this kind of navigation through the volume data set is generally very difficult, but may be facilitated by means of the valve model of the heart valve, because this model generally defines 2D sections within the annulus, each section corresponding to a morphological or physical feature of the heart valve, for example a leaflet, part of a leaflet, an area of regurgitant flow, etc. According to the invention, this information is projected into the volume, where it may be used to facilitate navigation through the volume data sets by a user. This is done in particular by projecting the valve model into the 3D or 4D volume data set along a projection direction to define a volume-of-interest. The volume-of-interest may be a cylinder having the area enclosed by the closed curve of the valve model as base. In case the closed curve is a circle, the cylinder will be a right circular cylinder; evidently it may have other shapes depending on the shape of the closed curve representing the annulus of the valve. Preferably, the centre of gravity of the base of the cylinder may correspond to a central point of the valve model, and may be aligned with the centre of gravity of the heart valve. In case of a circular cylinder, the cylinder axis, which crosses the centre point of the circle forming its base, is aligned with the centre of gravity of the heart valve. In a preferred embodiment, the height of the cylinder is the complete height of the 3D or 4D volume data set. In another embodiment, the valve model of the heart valve is projected only up to a set height. The projection may be in one direction or in both directions starting from the main plane of the annulus.

The volume-of-interest therefore describes and comprises a 3D volume above and/or below the heart valve, in particular the region into which the valve leaflets may move during a heart cycle. The volume-of-interest includes at least one 3D region, which is defined as being the projection of a 2D section within the valve model. A 2D section may for example define an area corresponding to a leaflet or another morphological or physiological feature of the heart valve. The corresponding 3D region is the projection of such a 2D section along the projection direction. In other words, the volume-of-interest includes at least one sub-volume, termed 3D-region, which also has the shape of the cylinder, not a circular cylinder, wherein the base of this sub-cylinder is a section corresponding to a morphological or physical feature. In a preferred embodiment, such a 3D-region may have the shape of a slice of pie, the pie being the volume-of-interest.

When visualising the volume-of-interest, a user may easily confuse one leaflet with another, or with further structures like papillary muscles. Therefore, the invention proposes labelling the voxels within the volume-of-interest, according to the 3D region to which they belong, wherein each 3D region is defined by the projection of a 2D section corresponding to a morphological or physiological feature of the heart valve. For example, the valve model may comprise one or several 2D sections, each 2D section corresponding to a leaflet. The 2D section may for example be a segment of a closed curve representing the annulus. If the valve model is then placed at the position and orientation of the annulus within the 3D volume data set or a frame of the 4D volume data set, and is then projected into the volume, the 2D sections get projected into the corresponding 3D region into which each leaflet represented by the sections may move during a heartbeat. Therefore, each leaflet may mainly move within its own 3D region within the volume-of-interest. According to the invention, each of the voxels in the at least one 3D region is labelled with label indicative of the morphological or physiological feature of the corresponding 2D section, for example a label indicating the particular heart valve. Thereby, the user may now navigate through the volume-of-interest within the 3D or 4D volume data set - preferably in all frames of a 4D volume data set - and may find voxels labelled according to the leaflet to which they will likely belong.

In useful embodiments, the valve model and in particular the projection direction is adapted to the position of the heart valve in each 3D frame of a 4D volume data set. In case the valve model is or is based on the 3D valve model, the 3D valve model is adapted over time, and is fitted to the annulus as it moves over a heart cycle. In case the valve model is based on a topology-based 2D model, the topology-based 2D model may be placed in the main plane of the annulus in each frame, for example as identified on the segmented heart valve. The projection direction is determined as described below. In useful embodiments, the placement of the valve model and the projection direction may be identified by using a 3D valve model of the annulus, which is positioned and oriented within the 3D or 4D volume data set at the position and orientation of the annulus of the heart valve, and which may be derived from the 3D or 4D volume data set, in particular from a segmentation of a heart valve, by an automatic or semi-automatic fitting algorithm.

According to an embodiment, the method comprises a further step of (e) providing a visualisation of the 3D or 4D volume data set of the heart valve, the visualisation comprising information from the projected valve model, in particular wherein voxels having different labels are visualised differently. The visualisation may for example be a rendering of the 3D or 4D volume data set, in case of a 4D volume data set preferably a dynamic rendering over at least one heart cycle. In the visualisation, the different 3D regions within the volume-of-interest are preferably visualised differently, so that the user may be always aware of the anatomical region he is navigating through, when viewing the visualisation.

According to an embodiment, the voxels in the at least one 3D region of the volume-of-interest are represented in a colour depending on their label. In useful embodiments, at least two 3D regions of the volume-of-interest are represented in different colours. For example, the 3D regions belonging to different leaflets may be coloured differently. This colour coding allows a user to be always aware of the leaflet region he/she is navigating through. For example, the 3D-regions may be coloured in one of red, green, yellow, magenta, orange or blue, or any other colour. Since it is sometimes hard to determine where exactly the leaflets meet in the centre of the heart valve, it is possible to leave a central 3D region unlabelled. In the valve model, this may be represented by a central 2D section which does not correspond to any particular morphological or physiological feature of the heart valve, but which is simply left "empty"; in other words, the 2D sections of the valve model do not fill the complete surface enclosed by the closed curve, but a central region is left undefined. Thus, the 3D region defined by the projection of this central section may not be labelled and may not be represented in any particular colour, but in a neutral colour. According to another embodiment, when representing or visualising voxels having different labels in different colours, there may be a soft transition between different colours in the visualisation, at least in some regions. This is in particular useful for a central region around the central axis of the volume-of-interest, in particular around the cylindrical axis. In embodiments where there is a hard transition from colour A to colour B at this axis, this may lend much importance to the central axis, although it in itself is not that relevant. Therefore, one may define a central region, e.g. a 3D region around the central axis, in which the shading of the visualisation will softly transition from one colour to the next, for example with a grey scale in between (colour A - grey scale - colour B). Alternatively, the 3D region around the central axis may be represented in a neutral colour.

According to a preferred embodiment, the visualisation includes visualising a 2D cut plane through the 3D or 4D volume data set. The 2D cut plane may be generated from the 3D or 4D volume data set by means of MPR. Preferably, the 2D cut plane may be oriented in any user-selected orientation through the volume data set. Thus, also the 3D regions of the volume-of-interest may be intersected at any angle. However, by visualising the voxels from different 3D-regions differently, the user is aided in finding his/her orientation within the volume data set. Preferably, all voxels in a 3D-region are coloured in the same colour, corresponding to the respective 2D section in the valve model. All 2D cut planes at any position and orientation through the volume data set, and preferably in any one of a sequence of frames, can display the corresponding colour, so that the location of a specific morphological or physiological feature of the heart valve is indicated. Thereby, anatomical knowledge is transformed into a colour-coding, which allows the user to be always aware of the leaflet region which he/she is navigating through. One can thereby navigate through the structures in the image without having to switch between image information and model information.

Representing the voxels in different colours may be implemented in various ways. According to one implementation, the image of the 2D cut plane is first generated, and then a colour-shading is projected on top of the image, as if the user was viewing the image through a "coloured glass". In another embodiment, the visualisation of the 2D cut plane already includes the colouring, which is associated with each voxel by means of the label of each voxel. Thus, when the 2D cut plane is generated, the voxels are already given their shade, according to the pixels of the image are already given their colour-shading, related to the respective label.

Anatomical structures outside of the volume-of-interest may remain unshaded. Alternatively, structures outside of the volume-of-interest, or outside of the volume-of-interest plus a certain margin, may not be represented at all. In other words, the volume-of-interest may also be used to "cut free" the heart valve, in order for it to be even better visualised.

The invention is not limited to visualising 2D cut planes, but a colour-coding scheme may also be used when visualising the 3D or 4D volume data set in a different way, for example by means of volume rendering or surface rendering. Volume rendering in particular means a technique used to visualise a three-dimensional image data set, and employs parameters like threshold, opacity and contrast, which may advantageously be adjusted "live" by the user, i.e., with immediate effect where the user is watching the rendering. The visualisation may also be a surface rendering, in particular a surface rendering of the segmented heart valve. In a further embodiment, the visualisation is a surface rendering of a surface 3D valve model of the heart valve, for example the 3D valve model described herein.

In case of a 4D volume data set, the visualisation in which the different 3D regions are represented differently, in particular with different colour-shading, is preferably dynamic, i.e., the visualisation is done for the complete 3D image clip, at least for a sequence of 3D images covering at least one heart cycle. Thereby, the user can analyse the dynamics of the heart valve while profiting from the anatomical information propagated through the 3D volume data set by means of the projection of the different 2D sections.

According to an embodiment, the voxels which are outside of the volume-of-interest are not visualised in the visualisation. This is a useful embodiment, because orientation within the data set may be further facilitated by cutting away those regions which are not of interest in the analysis of the valves. Voxels "outside of the volume-of-interest" may either be understood in the literal sense, or it may mean outside of the volume-of-interest with a certain margin, for example a user-defined margin of for example 3-15 millimetres, so as to cover also the neighbouring regions of the valve. In an alternative embodiment, voxels outside of the volume-of-interest are visualised differently, for example in a neutral colour, or in a lighter shade than the voxels within the volume-of-interest.

According to an embodiment, the valve model comprises a plurality of commissure points positioned along the circumference of the closed curve, wherein each commissure point defines a starting point of a commissure line between two leaflets of the heart valve, and wherein at least one section is partly delineated by at least one commissure line, and corresponds to a leaflet. The commissure lines thus may define a separating line between two adjacent leaflets of the heart valve, or the edges of the leaflets. The commissure points are located where the leaflets of the heart valve meet at their junction with the annulus. Therefore, the commissure points may be used to define the 2D sections corresponding to the different leaflets. The end point of the commissure line may be a central point of the valve model, e.g. its centre of gravity or the centre of a circle or ellipse representing the annulus. It may also be another commissure point, especially in case there are only two leaflets. It may also be another point on the valve model which has been identified e.g. on the segmentation of the heart valve as the end point of the commissure line. The commissure line itself may be a straight line between the starting point and the end point. It may also be a curved line, e.g. spanned by several points on the valve model. Thus, by connecting the commissure points with a central point or other points by commissure lines, the leaflets of the heart valve may be represented in the valve model in a simplified manner. In one embodiment, a straight line extends from each commissure point towards the centre of the closed curve representing the annulus, in particular the centre of a respective circle representing the annulus. Thereby, the valve model is divided into 2D sections like a pie chart, each section representing a leaflet. However, in other embodiments, in particular with only two leaflets, the line extending from the commissure points may have a different shape, which more closely resembles the actual anatomy of the valve. Further, since it is often difficult to define the heart valve exactly at its centre, i.e., along the cylindrical axis, in some embodiments a central region of the valve model is not assigned to any particular section, but is left undefined, and is then represented in a neutral colour. Thus, the centre of the valve model may include an inner circle as the central region, which may be concentric to the closed curve. Further, the commissure points may describe a subtle structure that can be identified by two anatomical landmarks, for example the axis of corresponding papillary muscles and the commissure chordae which have a specific fan-like configuration.

The commissure points on the valve model and the position of the commissure points in the 3D or 4D volume data set, in particular on the segmented heart valve, are preferably related to each other by a mapping function, such that both have the same relative position around the annulus, and/or the same relative position with respect to a reference point of the heart valve. The reference point may for example be the centre of gravity of the heart valve. This applies in case the valve model is a 3D valve model as described herein. It also applied in case the valve model is or is based on a topology-based 2D model as described herein.

According to an embodiment, a 2D section of the at least one 2D section within the closed curve corresponds to a physiological feature, wherein preferably the physiological feature is a flow phenomenon. Thus, the topology-based 2D model may be used to also define physiological features of the heart valve in a standardised manner, in particular projected onto the 2D plane of the topology-based 2D model. For example, certain areas where a blood flow phenomenon occurs may be represented as 2D sections on the topology-based 2D model. Such flow phenomena may for example be an area of regurgitant flow. If this is represented in a different way in a visualisation of the 3D or 4D volume data set, it may provide valuable information to the physician as to the exact position of the valve prolapse, thus assisting the planning of a surgery. In another embodiment, the flow phenomenon may also be a blood flow from a neighbouring valve, which the user wants to exclude from the visualisation of blood flow in the 3D or 4D volume data set. In this case, a respective section can be defined automatically or by the user on the topology-based 2D model. When projecting the model into the 3D or 4D volume data set, the 3D region relating to this section of undesired flow measurements may be labelled as an area where no flow phenomena are to be visualised. In a next step, the user may choose a visualisation of the 3D or 4D volume data set, in which flow information is included, in particular flow information from Doppler ultrasound measurements. The 3D region relating to the 2D section of undesired flow measurement may be excluded from the flow visualisation. In other words, in spite of the Doppler measurements which are available for this 3D region, the measurements are not displayed to the user. Thereby, the user can concentrate on the Doppler measurements showing regurgitant flow from the heart valve, and which are important for the clinical questions at hand, without distraction from undesired flow phenomena.

According to an embodiment, the valve model is based on a 3D valve model, which is positioned and oriented within the 3D or 4D volume data set at the position and orientation of the annulus of the heart valve, and wherein the projection direction extents at least approximately perpendicular to a plane fitted to the 3D valve model of the annulus. In this context, "at least approximately" means up to ± 10%, i.e., at an angle between 81°and 99° to the fitted plane. Examples of how to generate a 3D valve model of the annulus from the 3D or 4D volume data set of the heart valve are explained herein above. This method may be applied to each frame within a video clip (4D volume data set). Alternatively, the 3D valve model of the annulus may be generated for one frame of a video clip, and the model may be tracked through the series of frames for example by following a number of anatomical landmarks from one frame to the next.

In order to project the 2D sections from 3D valve model into the 3D or 4D volume data set, the 3D valve model may first be projected into a plane, in particular a plane which is perpendicular to the projection direction. The 3D valve model is not necessarily limited to a 2D plane, but may have a three-dimensional shape. Therefore, one may calculate the orientation of a plane which has the least quadratic distance to a non-planar surface enclosed by the closed curve of the valve model. Therein, for example a set number of points, such as 5-30, preferably 10-20, points are distributed over the non-planar surface of the 3D model. Then, a minimisation algorithm is performed in order to find a plane which minimises the sum of the squares of the distances from each point to the plane. This plane is also referred to as "fitted plane" and may correspond to the main plane of the annulus. In alternative embodiments, the fitted plane may be determined only from points on the outer circumference of the 3D valve model (the 3D ring), preferably also by minimising the sum of squares of the distances from the outer circumference to a corresponding flat ring. The flat ring then defines the plane. The 3D valve model may be projected into this plane, together with any 2D sections comprised therein, to result in a 2D valve model. This 2D valve model is then the valve model that is projected into the 3D or 4D volume data set.

In other embodiments, the orientation of the fitted plane is taken as the orientation of the topology-based 2D model, and the topology-based 2D model is the valve model that is projected into the 3D or 4D volume data set. The topology-based 2D model is then positioned at the position of the 3D valve model, thus it is positioned over the annulus of the heart valve. Also, any commissure points defined on the topology-based 2D model are positioned as close as possible, for example calculated by a minimisation algorithm, to the commissure points as defined in the 3D or 4D volume data set, or on the 3D valve model or on the segmented heart valve. Thereby, a projection direction which extends from the area of the heart valve into the heart can be identified, and the projection of the topology-based 2D model can be performed as accurately as possible.

According to an embodiment, the visualisation is a dynamic 4D visualisation of the heart valve, and the projection direction of the valve model is adjusted according to the movement of the heart valve. In this embodiment, the projection direction is calculated for each frame within a 4D volume data set. This may be done by using a dynamic 3D valve model of the annulus, obtained for example by tracking the 3D valve model from one frame to the next. Then, a plane may be fitted through the 3D valve model for each frame within a 3D video clip of the heart valve. Thereby, the labelling of the voxels in the 3D regions may be adjusted to the movement of the heart over the heart cycle, and the visualisation of the moving heart may be dynamically adapted to the position of the heart valve during the heart cycle. This greatly facilitates analysis of the heart valve.

According to an embodiment, the volume-of-interest extends into the 3D or 4D volume data set up to a set height along the projection direction, preferably a height which is proportionate to a dimension of the valve model. In other words, the height of the volume-of-interest is not the complete height of the volume data set. This has the advantage that the visualisation comprising information from the projected valve model may be limited to the area around the valve, so as not to distract the user. In particular, in visualisations where different 3D regions are represented in different colour, too much colour can be distracting to a user.

This set height of the volume-of-interest may either be proportionate to a dimension of the valve model. For example, it may correspond to the widest diameter of the closed line representing the annulus, which may be the diameter of a circle representing the annulus. It may also be proportional to this diameter, but multiplied by a pre-determined factor, for example between 0.5 and 1.5. In other embodiments, the height of the volume-of-interest may be set by a user. Thereby, the user can adapt the visualisation specifically to his/her requirements. In particular, in embodiments where the voxels outside of the volume-of-interest are not visualised, the user can thereby determine exactly the size of the data that should be visualised and analysed. But also where the visualisation of the volume-of-interest includes colour-coding of the voxels in different 3D regions, it is useful to limit the colour-coding to a certain neighbourhood of the valve. Thus, the height of the volume-of-interest may be set either manually or automatically. It may be also be first set automatically, for example to a height in proportion to the dimension of valve model. The user may then be given the chance to manually adjust the height of the volume-of-interest.

According to an embodiment of the method of the invention, a topology-based 2D model of the heart valve is used, which is preferably a highly simplified valve model represented on a plane, i.e., it is 2D-dimensional (2D) in the sense that all elements of the topology-based 2D model can be represented in 2D coordinates. The topology-based 2D model preferably comprises a closed curve representing the annulus. The closed curve preferably has a standardized shape, for example a circle, ellipse, polygon, or polygon with rounded edges. The size of the closed curve may be standardised, regardless of the actual size of the heart valve annulus. In other embodiments, the size of the closed curve is proportional to the actual size, e.g. the widest diameter, of the annulus of the heart valve. Thus, the topology-based 2D model is preferably a standardized, simplified visualisation of the main elements of the heart valve, in particular the annulus and the leaflets. The area within the closed curve may comprise at least one 2D section, wherein the at least 2D section represents a morphological or physiological feature of the heart valve. An advantage of the topology-based 2D model is that it provides a simplified visualisation of the characteristics of the heart valve. Thereby, the topology-based 2D model may be used to easily compare different heart valves with one another.

In order to be able to adjust the topology-based 2D model, preferably a 3D visualisation of the 3D valve model from which it is derived, together with at least part of the 3D or 4D volume data set of the heart valve is also displayed to the user. In preferred embodiments, the segmented heart valve is displayed in a volume or surface rendering, and the 3D valve model is overlaid therewith. This visualisation is preferably dynamic, i.e., it follows the movement of the heart valve across a time period of for example one heartbeat. Since the 3D valve model is directly related through a mapping function to the topology-based 2D model, any changes made in the topology-based 2D model may be directly transferred to the 3D valve model, and included in the visualisation displayed to the user. Vice versa, in some embodiments the user can also make amendments to the 3D valve model, for example by moving, deleting or adding a commissure point, and this amendment will be immediately transferred through the mapping function to the topology-based 2D model. Thereby, the user is able to make adjustments to the topology-based 2D model, in order to adapt it to the actual heart valve.

According to another aspect, the invention relates to a method for visualising a 2D cut plane of the 3D or 4D volume data set enriched with the morphological information from the valve model. In particular, the voxels in the volume-of-interest are duly labelled according to the respective label of the 2D section. The rendering properties of the pixels in the 2D cut plane are set depending on the labels associated with the voxels of the volume-of-interest that are intersected by the 2D cut plane. The visualisation may be performed on the user interface, which may be configured to display a visualisation of the 3D or 4D volume data set together with information from the projected valve model. The visualisation environment may be a three-dimensional visualisation environment or a virtual reality environment. By "virtual reality", any computer-generated visualisation providing a truly three-dimensional experience of the depicted structure is meant. Accordingly, the virtual reality (VR) environment of the invention provides in particular visual feedback, but may also allow other types of sensory feedback, such as auditory. The VR environment may also be an augmented reality environment, wherein the user still sees the real environment, but with the VR objects (e.g., the volume rendering) overlaid or superimposed on the reality objects, or a mixed reality, in which real-world objects are superimposed on a virtual scene. The visualisation environment further preferably allows the user to make adjustments to the valve model and optionally the topology-based 2D model, e.g. by a "drag-and-drop" functionality. The visualisation environment may be used to view and analyse the heart valve, in particular for planning interventions and/or determining the correct size, shape and position of implant to be implanted in future interventions.

As explained above, the visualisation of the 3D or 4D volume data set of the heart valve may - in addition to a 2D cut plane - include a rendering, such as a volume rendering or surface rendering, of the segmented heart valve, preferably overlaid with the valve model, in particular the 3D valve model. The corresponding topology-based 2D model may be visualised next to the rendering. Thereby, the user may adjust the valve model to the anatomy of the heart valve on the visualised rendering, and may immediately see any changes in the colour shading of the 2D cut plane. The user may preferably further adjust the orientation of the 2D cut planes through the 3D or 4D volume data set. The visualisation is preferably dynamic, i.e. it follows the movement of the heart valve across a time period of for example one heartbeat.

According to an embodiment, the method comprises a further step of receiving user input data for creating or adjusting at least a 2D section within the valve model via a user interface. Preferably, the user interface comprises a display configured to display the visualisation, and one or more user input devices, which may be operated by a user to provide user input. Such user input devices may be a computer mouse, joystick, touchpad etc. Preferably, the display may be a touch sensitive display. In this case, no additional user input device is necessary. The user interface may comprise a processor and a storage for storing information. Preferably, the user interface is a computer or is part of a computer.

In embodiments where the valve model is or is based on a 3D valve model as described herein, the 3D valve model is preferably visualised overlaid on a visualisation of the 3D or 4D volume data set, for example a volume rendering thereof, and the user may amend the 3D valve model to better fit the anatomy of the heart valve. According to a preferred embodiment, a topology-based 2D model, which may be derived from the 3D valve model as described herein, is also displayed to the user, for example next to the visualisation of the 3D or 4D volume data set or on the same screen or another screen, and any adjustment of a 2D section made on the topology-based 2D model by the user is transferred to the 3D valve model. Thereby, the user may edit the topology-based 2D model - instead of the 3D valve model - to better fit to the morphological or physiological features of the heart valve. According to a preferred embodiment, any adjustments made by the user are immediately visible in the visualisation of the 3D or 4D volume data set. For example, if the user edits a 2D section, for example by moving a commissure point around the closed curve, adding or deleting a commissure point and thereby adding or deleting a leaflet, the mapping of the topology-based 2D model to the 3D valve model displayed overlaid on the 3D or 4D volume data set is immediately adjusted accordingly. Therefore, also the 3D regions within the volume-of-interest change, and this is immediately visualised, for example by changing the colour-shading. This has the advantage, that the user can immediately profit from a better adaptation of the topology-based 2D model to the morphology of the actual heart valve, which will aide in the analysis of the 3D or 4D volume data set.

According to a further embodiment of the present invention, the user input comprises instructions to modify a position of at least one commissure point of the valve model. For example, the user may virtually grasp the commissure point to be modified and move it to a desired position on the annulus (or closed curve of the valve model). The user may preferably modify the commissure point in a 3D visualisation of the valve model, in particular the 3D valve model, overlaid on a visualisation of the heart valve, e.g. the segmented heart valve. The corresponding commissure point in the topology-based 2D model, whose position is not set directly by the user, automatically and simultaneously adjusts its position to match the position of the commissure point set directly by the user. Preferably, the commissure points and the commissure lines are displayed on both the topology-based 2D model and on a visualisation of the 3D valve model overlaid on the (segmented) heart valve. This may provide an opportunity to verify that commissure points are well placed in different images of the 3D or 4D volume dataset.

According to a still further embodiment of the present invention, the user input comprises instructions to add a new commissure point and/or to delete a commissure point. This is advantageous because different valves may have different numbers of leaflets, and even each person may have a different number of leaflets e.g. in their tricuspid valve. Therefore, the valve model may be adapted to the specific heart valve under inspection. For example, the user may add a further commissure point by clicking on the closed curve of the valve model. A new commissure line may be automatically created extending from the new commissure point to the centre of the valve model. Accordingly, a further leaflet may be depicted by the valve model. In an analogous way a commissure point may be deleted as well as the commissure line defined by the deleted commissure point. As a result, the valve model may have one less leaflet, and one less 2D region.

According to a further embodiment, the method further includes labelling the 2D sections, in particular with labels corresponding to the leaflets defined by the at least one commissure line. The labels may correspond to generally used indications of the respective leaflets. For example, the tricuspid valve may comprise an anterior cusp, a posterior cusp and a medial cusp. The aortic valve may comprise a left coronary cusp, a right coronary cusp and a non-coronary cusp. The leaflets may be labelled according to the nomenclature proposed by Rebecca T. Hahn, T. Weckbach, Thilo Noack, Nadira Hamid, Mitsunobu Kitamura, Richard Bae, Philipp Lurz, Susheel K. Kodali, Paul Sorajja, Jörg Hausleiter, Michael Nabauer, "Proposal for a Standard Echocardiographic Tricuspid Valve Nomenclature", JACC: Cardiovascular Imaging, Volume 14, Issue 7, 2021, Pages 1299-1305, ISSN 1936-878X, in case of the tricuspid valve, which is included herein by reference. The label of the respective leaflet may be used to label the 2D sections of the valve model. When the valve model is projected into the volume, the various 2D sections representing the different leaflets are converted into 3D regions, which are labelled accordingly. The visualisation of a cut plane, in which the 3D regions having different labels are represented in different colours, may thus be colour-shaded according to the label of the respective leaflet. The visualisation may include a caption, in which the respective colours are linked to the labels of the respective leaflets. Thereby, the labelling of the leaflets is transformed into a colour-coding, which allows the user who is viewing the heart valve on various cut planes through the 3D or 4D volume data set, to be always aware of the leaflet region he is navigating through.

According to a still further embodiment of the present invention, the method further comprises visualising a flow phenomenon on a 3D visualisation of the heart valve based on flow information, identifying a location on the valve model corresponding to a location of the flow phenomenon, and providing a 2D section or marker at the identified location.

In the state of the art, a projection of the leakage position on the valve is not intuitive for the user. The affected area for the procedure cannot be marked easily and must be estimated in the current state of the art. Currently, many of these procedures are performed without specific planning, increasing the time on the operating table as well as the abort rate due to incompatibility of the method or device. If image-based planning is performed, it is done in a fully manual way, taking up valuable physician time and showing a high variability and error-proneness. In the present embodiment, an easy way is provided to indicated a leakage flow in the valve model in the form of a marker or a 2D section, which is labelled accordingly, and to project this information back into the 3D or 4D volume. Accordingly, the planning of an intervention may be facilitated and made more accurate. The flow phenomenon may be derived from Doppler information included in the 3D or 4D volume data set. Accordingly, flows (e.g., of blood) may be visualized. The Doppler information may be in the same coordinate system as the segmented heart valve.

Accordingly, the flow phenomenon may be visualised at the correct position relative to the (segmented) heart valve or the 3D valve model. This 3D position of the flow phenomenon in the visualisation of the segmented heart valve may then be mapped to the valve model. This may be done by points of reference existing in both the visualised segmented heart valve and the valve model. Such points of reference may be some or all commissure points and/or a centre of the heart valve. Then, the position of the flow phenomenon is known in the valve model and may be indicated by a marker or a 2D section, for example an area of pre-defined size and shape around a marker. Alternatively, the area of the 2D section may be calculated based on the Doppler information included in the 3D or 4D volume data set. The location and size of the area may then be projected on the topological-based 2D model for easier localization in procedure planning. The local extent of leakage may be indicated by contour lines or a heat map. Accordingly, an intervention may be planned precisely in advance.

In an embodiment, a regurgitant flow may be dynamically indicated on the valve model for the period in which the valve is typically closed (mitral, tricuspid: systole, pulmonic, aortic: diastole). Further, the location of the area of leakage may be indicated in the valve model. The 2D section indicating a flow phenomenon in the valve model may be dynamic. That is, the 2D section indicative of the flow phenomenon may change depending on the heart cycle currently depicted by the visualisation of the segmented heart valve. Accordingly, the flow phenomenon may be inspected by the user in different positions of the heart valve.

According to a further embodiment, the 2D section includes a marker including a computer graphical representation of flow phenomenon indicative of the magnitude of the flow phenomenon, for example as iso-lines, vector field, streamlines and/or a colour map. In other words, the scale and the area size of the leakage may be displayed on the valve model, and preferably on the topology-based 2D model. That is, a magnitude of leakage through a heat map or contour graph (isolines) may be displayed in the visualisation of the segmented heart valve and/or the topology-based 2D model. Therefore, the meaningfulness of the valve model may be even further increased by indicating a location, extent, and direction of the flow phenomenon (e.g., regurgitant flow).

According to a still further embodiment of the present invention the method further includes receiving user input indicative of a location or 2D region on the valve model, in which no marker (i.e., no flow phenomenon) should be displayed. In other words, the user may define an exclusion area in the valve model. For example, if the heart valve under inspection is provided adjacent to a second heart valve, there may be side stream induced by the second heart valve which do not describe the function of the heart valve under inspection. Therefore, it may be helpful to exclude some areas of the valve model from displaying flow phenomenon.

According to another aspect, the invention provides a computer program comprising program code instructions which, when executed by a processor, induce the processor to carry out the inventive method. The computer program may be in any code, in particular a code suited for computer graphic applications.

In a further aspect, the invention is directed to a computer-readable medium comprising an above-defined computer program. The computer-readable medium may be any digital data storage device, such as a USB-stick, hard disk, CR-ROM, SD card or SSD card. Naturally, the computer program need not be stored on such a computer-readable medium to be supplied the customers, but may be downloadable via the internet.

According to another aspect, the invention provides a system for analysing a heart valve including a user interface configured to receive user input, and a control unit configured to execute the above method. Any features and advantages of the system and the computer program also applied to the method and vice versa.

The system may be included in the acquisition modality, in particular an echocardiography system. Accordingly, the valve model may be instantly generated during acquisition of the 3D or 4D volume data set. Alternatively, the system may be provided centralized and may be connected to a database in which a plurality of 3D or 4D volume data sets are stored. In this case, a valve model may be created for each data set and may be used in research and/or for future intervention planning.

According to an aspect of the invention, a topology-based 2D model mapped bidirectionally to a 3D valve model of the heart valve that can serve as both an edit control ("valve joystick") and a parametric display ("valve dashboard") of the valve is provided. For the valve joystick the user input is mapped to the topology model on the 4D valve segmentation. The user can add or delete commissure points on the model and change their relative position. These edits are displayed on a 3D valve model based on the segmented heart valve and tracked over time.

### BRIEF DESCRIPTION OF THE DRAWINGS

Useful embodiments of the invention shall now be described with reference to the attached figures. Similar elements or features are designated with the same reference signs in the figures. In the figures:
Fig. 1 is a schematic diagram of a visualisation of a 3D valve model of a heart valve and a topology-based 2D model according to an embodiment of the present invention,
Fig. 2 is a schematic diagram of a visualisation of a 3D valve model of a heart valve and a topology-based 2D model according to an embodiment of the present invention,
Fig. 3 is a schematic diagram of a plurality of topology-based 2D models according to an embodiment of the present invention,
Fig. 4 is a schematic diagram of a topology-based 2D models being adjusted according to an embodiment of the present invention,
Fig. 5A is a schematic diagram of a visualisation of a 3D valve model of a heart valve and a topology-based 2D model according to an embodiment of the present invention,
Fig. 5B is a schematic diagram of a visualisation of a 3D valve model of a heart valve and a topology-based 2D model according to an embodiment of the present invention, and
Fig. 6 is a schematic diagram of a user interface according to an embodiment of the present invention,
Fig. 7 is an illustration of the projection of the topology-based 2D model into the 3D or 4D volume data set according to an embodiment of the invention,
Fig. 8 is an illustration of a cut plane.

### DETAILED DESCRIPTION OF EMBODIMENTS

Throughout the figures, the same or corresponding features/elements of the various embodiments are designated with the same reference numbers.

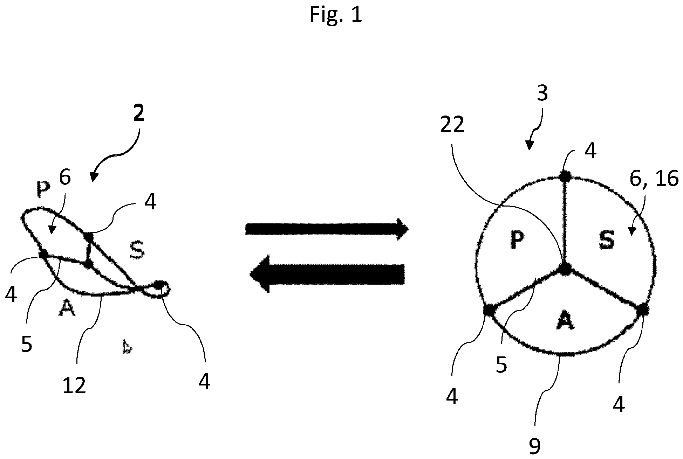

Fig. 1 is a schematic diagram of a visualisation of a 3D valve model 2 of a heart valve, and a topology-based 2D model 3 according to an embodiment of the present invention. On the left side in Fig. 1, the visualisation of a valve model, in this case a 3D valve model 2, is depicted. This may usefully be overlaid on a visualisation, e.g. volume rendering of the segmented heart valve (not shown). On the right side of Fig. 1, the topology-based 2D model 3 is depicted. The 3D valve model may be derived from the heart valve segmented from a 3D or 4D volume data set. The topology-based 2D model 3 is created from the 3D valve model e.g. by projecting it onto a 2D plane, as explained herein above. The heart valve has three leaflets 6. The topology-based 2D model 3 includes three commissure points 4 on a closed curve 9 and three commissure lines 5 connecting the commissure points 4 with the centre point 22 of the - in this case circular - closed curve 9. The three commissure lines 5 together with the respective segments of the circle 9 representing the annulus define three 2D sections 16 in the topology-based 2D model 3, each corresponding to a leaflet 6. In other words, the commissure points 4 are connected with each other via the centre 22 of the topology-based 2D model 3. The three commissure points 4 and three commissure lines 5 are also present on the 3D ring 12 representing the annulus of the 3D valve model 2. The 3D ring 12 is the closed curve of the 3D valve model 2. The commissure points 4 may be adjusted by the user via a user interface 10, such that the commissure points 4 correspond to a junction of the borderline of the leaflets 6 with the annulus 12. Through drag-and-drop, the topology-based 2D model 2 can be adjusted to fit the individual anatomy of the heart valve (e.g., the leaflets 6) and the corresponding 3D valve model is displayed on the visualisation of a segmented heart valve. The following changes may be made to the commissure points 4:
a. Add / delete commissure points 4 on the topology-based 2D model based on morphology, and
b. Drag and drop commissure points 4 along closed curve 9. In this case, commissure points 4 on the 3D valve model will follow along the 3D ring 12.

The 2D sections 16 between commissure lines 5 are labelled with abbreviations of the respective valves according to standard anatomical nomenclature, in this case with letters "A", "S" and "P". Each section 16 represents one leaflet 6 of the heart valve. In addition, the mapping of the topology-based 2D model 3 to the 3D coordinates of the 4D volume data set is tracked through one complete heart cycle e.g. through the temporal tracking of the 3D ring 12 representing the annulus. The morphology nomenclature is mapped to the adjusted topology-based 2D model. The arrows in Fig. 1 show that an adaption of the commissure points 4 may be done in the topology-based 2D model 3 and in the visualisation of a segmented heart valve or the 3D valve model 2. The amendments are mapped to the respective other one thereof.

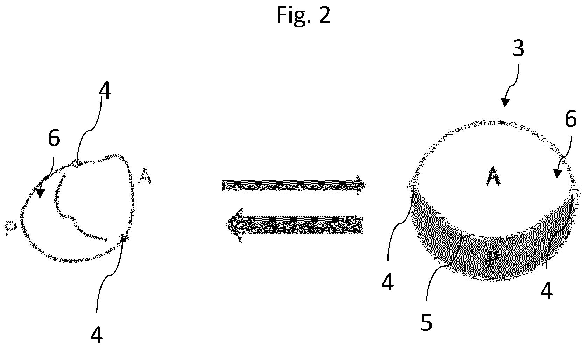

Fig. 2 is a schematic diagram of a visualisation of a 3D valve model 2 and a topology-based 2D model 3 according to another embodiment. The present embodiment essentially corresponds to the previous embodiment with the exception that another heart valve is depicted having only two leaflets. However, the procedure is the same as in the previous embodiment. Further, in Fig. 2 the commissure points 4 are directly connected to each other via a commissure line 5. In other words, the commissure line is not routed over a centre of the topology-based 2D model. In this case the commissure line may be adjusted by the user so as to fit to the borderline of the two leaflets 6 of the heart valve.

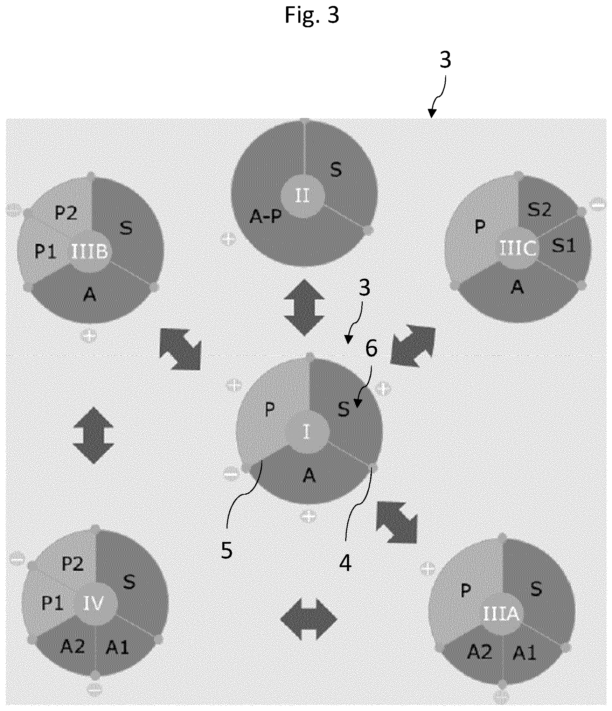

Fig. 3 is a schematic visualisation of a plurality of topology-based 2D models 3 according to an embodiment of the present invention. In the centre of Fig 3 an initial topology-based 2D models 3 is depicted. That is, the initial topology-based 2D model 3 is not adjusted to the real heart valve, but has initial settings, in this case three commissure points 4 and three commissure lines 5. The topology-based 2D models 3 surrounding the initial topology-based 2D model 3 in Fig. 3 are adapted topology-based 2D models 3. The topology-based 2D models 3 are created as in the previous embodiments. Accordingly, a user may add, delete and/or shift commissure points 4 so as to adjust the topology-based 2D model 3 to the real heart valve depicted in the visualisation of a segmented heart valve. Further, the labels of the leaflets 6 may be manually amended by the user. In each topology-based 2D model 3 in Fig. 3, the centre of the topology-based 2D model 3 is a circle coaxial to the circular closed curve 9 of the topology-based 2D model 3. Within the centre circle a tag is provided which is indicative of a type of heart valve. The 2D sections 16 of the topology-based 2D model are labelled with an abbreviation of the name of the respective valve, such as P1, Ps, A and S.

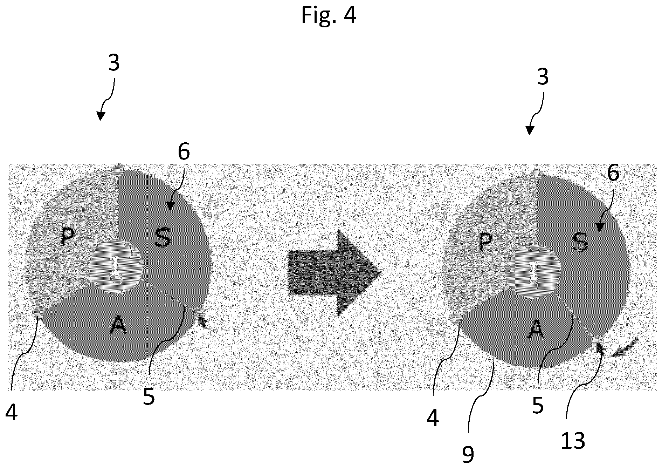

Fig. 4 is a schematic visualisation of a topology-based 2D models being adjusted according to an embodiment of the present invention. That is, on the left side in Fig. 4 the initial topology-based 2D model 3 is depicted. Then, the user may grab a commissure point 4 with a pointing device 13 and drag the commissure point 4 in the desired direction along the model annulus or closed curve 9 (refer to the small arrow in Fig. 4). The adjustment to the commissure point 4 is preferably automatically applied to the 3D valve model (not depicted in Fig. 4), and the 3D valve model is displayed on the 4D Echo clip (i.e., the visualisation of a segmented heart valve 2). The complete topology-based 2D model 3 can be rotated to fit the alignment of the Echo clip. Through "+" and "-" buttons around the model annulus, commissure points 4 can be added and removed in every sector of the topology-based 2D model 3 to fit the number and morphology of leaflets 6. Drag-and-drop of the commissure points 4 along the closed curve 9 adjusts the commissure points 4 on the Echo annulus 12. The leaflets 6 are labelled according to the nomenclature proposed by Hahn et. al. in case of the tricuspid valve. The selected morphology based on number and position of the commissure points 4 is mapped to the proposed nomenclature and a type is assigned in case of the tricuspid valve. The default type on initialization is the most common - Type I.

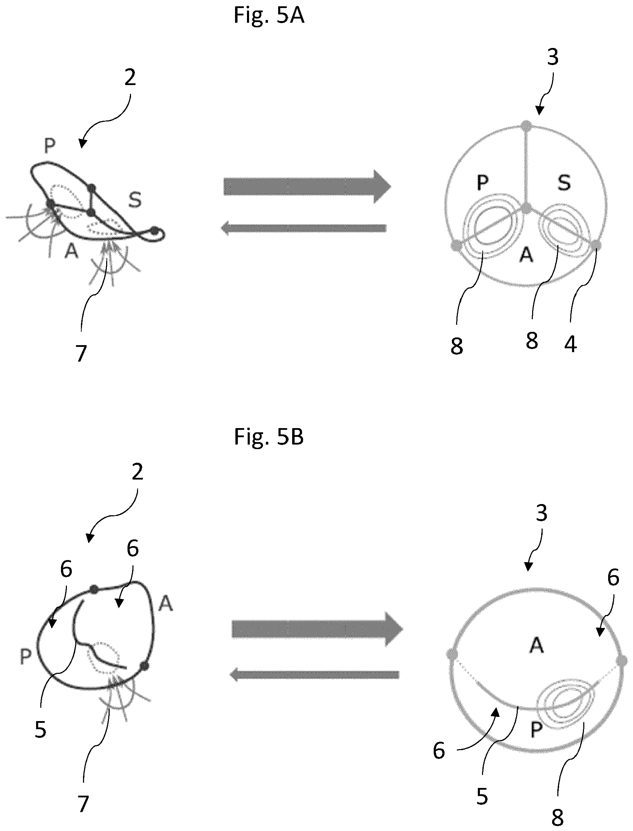

Fig. 5A is a schematic diagram of a visualisation of the segmented heart valve together with the 3D valve model 2 and a topology-based 2D model 3 according to another embodiment of the present invention. In the present embodiment, in the segmented heart valve 2 and a topology-based 2D model 3 at least one flow phenomenon 7 is additionally indicated. A parametric display is provided which projects a flow phenomenon from 4D Echo data, e.g. a 3D or 4D volume data set comprising flow information e.g. from Doppler ultrasound, to topology-based 2D model 3. Specifically, as an example of the flow phenomena 7,in the segmented heart valve 2, a regurgitation flow is dynamically displayed for the period during which the valve is typically closed (mitral, tricuspid: systole, pulmonic, aortic: diastole). Then, a location of the corresponding area of leakage 8 on the topology-based 2D model 3 is displayed. In addition, the area size of the leakage 8 is shown scaled on the topology-based 2D model 3. Further, a magnitude of leakage through a heat map or contour graph (isolines) is displayed on the projection. The visualisation of the flow phenomena 7 in the topology-based 2D model 3 is realized by a marker 8 which might be changed with respect to its visualisation properties. The marker also represents a 2D section of the topology-based 2D 3 which is labelled with a physiological feature, namely a flow phenomenon.

Fig. 5B is a schematic visualisation of a visualisation of a segmented heart valve and a topology-based 2D model according to an embodiment of the present invention. Fig. 5B corresponds essentially to Fig. 5A with the exception that the heart valve in Fig. 5B has only two leaflets.

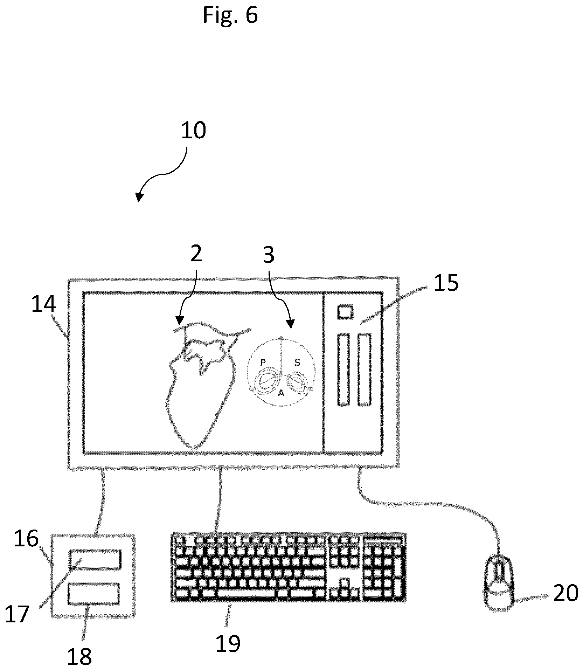

Fig. 6 shows a user interface 10 according to an embodiment of the invention. In this setting, the segmented heart valve 2 and the topology-based 2D model 3 are depicted on a conventional computer screen 14. The screen 14 may comprise a panel 15 of buttons and sliders allowing the user to tilt, zoom, move or otherwise manipulate the segmented heart valve 2 and/or the topology-based 2D model 3. Also in such a user interface 10, a 2D cut plane through the 3D or 4D volume data set may displayed, wherein at least one 3D region is depicted in a colour shading characteristic for the label of the respective 3D region (not shown). The display may be controlled by a computer 16, such as a PC, including a processor 17 and a hard disc 18. The user interface may have input tools such as a keyboard 19 and/or a mouse (i.e., a pointing device 20).

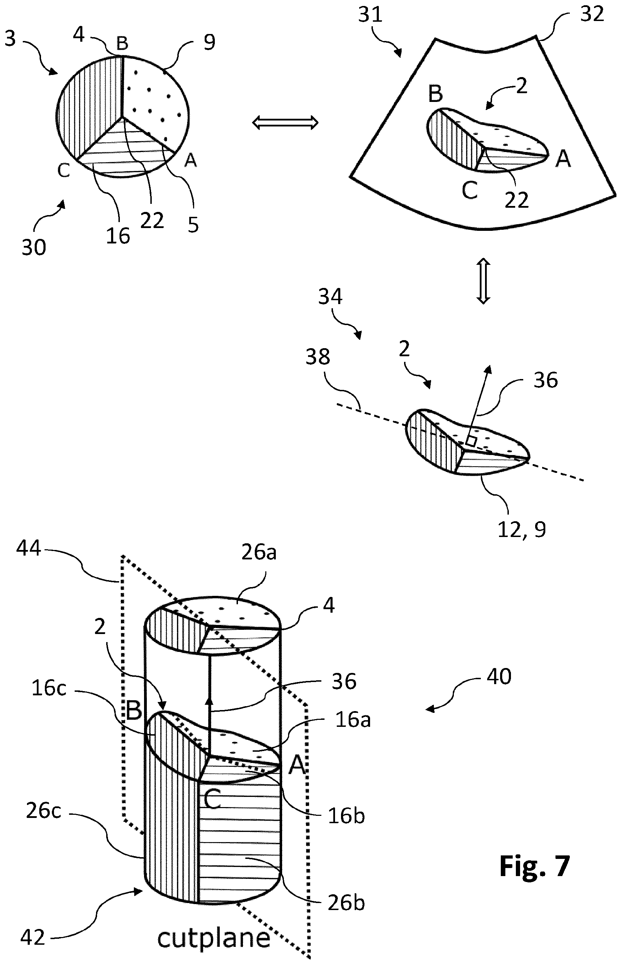

Fig. 7 illustrates the projection of a valve model into the 3D or 4D volume data set 32 according to an embodiment of the invention. At 30, a topology-based 2D model 3 is visualised. This particular topology-based 2D model 3 includes three commissure points 4, named A, B and C, on a closed curve 9 (in this case a circle), centred around a centre point 22. It thus comprises three 2D regions 16, each representing one leaflet, and each 2D section 16 is surrounded by a segment of the closed curve 9 and two commissure lines 5. The topology-based 2D model 3 is derived from a corresponding 3D valve model 2. In the visualisation 31, the 3D valve model 2 is visualised overlaid on a 3D echo clip 32, wherein the segmented heart valve is shown in a dynamic volume rendering view covering a time period of for example one or several heart cycles. For each frame of the 4D volume data set (3D video clip), the calculation illustrated at 34 is performed. Therein, the projection direction illustrated by arrow 36 is calculated. This is done by first finding a plane 38 which best fits the 3D valve model 2, wherein the 3D valve model 2 in particular comprises a three-dimensional ring 12 representing the annulus, and a three-dimensional surface enclosed by the ring 12. Preferably, the plane 38 is calculated to have the minimum square distance from this 3D surface. The projection direction 36 is then a vector perpendicular to this "average" plane 38, which represents essentially the main plane of the heart valve. This calculation is preferably repeated for each frame, i.e., for each shape and relative position of the 3D valve model 2 within the 3D or 4D volume data set of the heart valve, which is visualised at 31 as a 3D video clip 32.

Thus, the 3D valve model 2 can be projected onto the plane 38, together with all 2D sections comprised therein, preferably using a parallel projection along projection direction 36. This results in a projected 3D valve model. Alternatively, the topology-based 2D model 3 can be positioned in the plane 38 and aligned with the 3D valve model 2, for example by aligning the positions of the centre points 22. From this plane 38, the valve model, in particular the projected 3D valve model or the topology-based 2D model 3, is projected into the respective frame of the 4D volume data set 32 along projection direction 36, as illustrated at 40. Alternatively, the 3D valve model 2 is projected directly along the projection direction 36, without first generating a projected 3D valve model.

It can be seen in the illustration 40 how the projection of the 3D valve model 2 into the volume data set 32 results in a cylindrical volume-of-interest 42, which in this case has the complete height of the volume data set 32. The three 2D sections 16a, 16b, 16c are converted into 3D regions 26a, 26b, 26c, each shaped essentially like a slice of pie. Preferably, the voxels in the volume data set 32 within each of these 3D regions 26a, 26b and 26c receive a different label, according to the 3D region in which they are located. The different labels, which may translate into different colour-shadings, of each 3D region are illustrated as "dotted" for 3D region 26a, "horizontally ruled" for 3D region 26b, and "vertically ruled" for 3D region 26c. In other embodiments, the 3D valve model may be projected only along a height which is not up to the complete height of the volume data set 32, but for example up to a pre-defined multiple of the diameter of the 3D ring 12 or closed curve of the 3D valve model 3.

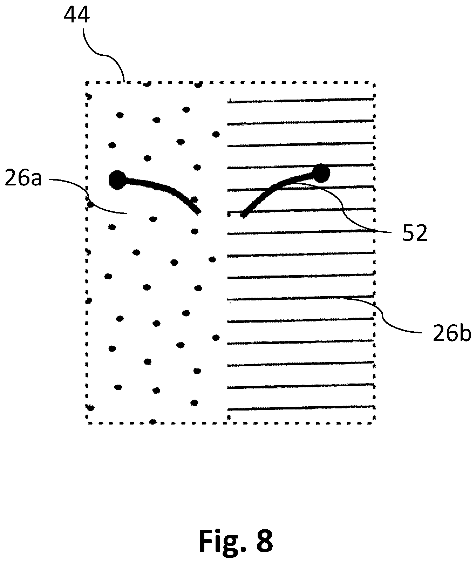

Fig. 8 illustrates how the method of the invention may be used to generate visualisations in which voxels from different 3D regions are represented in different colours depending on their label. As illustrated in part 40 of Fig.7, a plane 44 may be oriented in any orientation through the volume data set 32 and the volume-of-interest 42, which was generated by projection of the topology-based 2D model 3 along the projection direction 36 into the volume data set 32. The orientation of plane 44 may be adjusted by the user according to his/her needs, in order to obtain a good view through the volume data set. A corresponding cut plane 44 may be generated by means of multi-planar construction from the volume data set 32. The 2D cut plane 44, as illustrated in Fig. 8, may for example be visualised next to the visualisation of the volume data set 32. In the illustrated example, a cut through the valve 52 is visible on the 2D cut plane 44. Since the 2D cut plane 44 intersects two 3D regions 26a, 26b, namely two regions belonging to different leaflets, the areas belonging to the different 3D regions are illustrated in different colour shades: The 3D region 26a is shaded in one colour illustrated as dots and the 3D region 26b is shaded in another colour, illustrated by a horizontally ruled surface. This colour-shading is kept, even when the orientation of the cut plane 44 is changed by the user. Thereby, the user can navigate through the volume data set 32 more easily, since he is always aware of the leaflet region that he is navigating through by moving the position and orientation of the view plane 44.

The above discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the broader and intended spirit and scope of the present system as set forth in the claims that follow. Accordingly, the specification and drawings are to be regarded in an illustrative manner and are not intended to limit the scope of the appended claims.

## Claims

**1.** A computer-implemented method for analysing a heart valve (52) of a subject, the method comprising the steps of
(a) providing a 3D or 4D volume data set (32) of a heart valve (52) of a subject;
(b) providing a valve model (2, 3) of the heart valve (52), wherein the valve model (2, 3) is adjusted to the morphology of the heart valve (52), and
wherein the valve model (2, 3) comprises a closed curve (9, 12) representing the annulus of the valve, wherein the area within the closed curve (9, 12) comprises at least one 2D section (16), each 2D section (16) corresponding to a morphological or physiological feature of the heart valve (52); and
(c) projecting the valve model (2, 3) into the 3D or 4D volume data set (32) along a projection direction (36) to define a volume-of-interest (42), wherein the volume-of-interest (42) includes at least one 3D region (26), each 3D region (26) being defined by the projection of a corresponding 2D section of the at least one 2D section (16);
(d) labelling each of the voxels in the at least one 3D region (26) with a label indicative of the morphological or physiological feature of the corresponding 2D section (16).

**2.** The method of claim 1, comprising a further step of
(e) providing a visualisation of the 3D or 4D volume data set (32) of the heart valve (52), the visualization comprising information from the projected valve model (2, 3), in particular wherein voxels having different labels are visualized differently.

**3.** The method of claim 2, wherein voxels in the at least one 3D region (26) of the volume-of-interest (42) are represented in a colour depending on their label.

**4.** The method of claim 2 or 3, wherein the visualisation includes visualizing a 2D cut plane (44) through the 3D or 4D volume data set (32).

**5.** The method of any one of claim 2 to 4, wherein the voxels within a 3D region of the at least one 3D region (26) are excluded from the visualisation and/or are represented in a neutral colour.

**6.** The method of any one of claims 2 to 5, wherein voxels which are outside of the volume-of-interest (42) are not visualized.

**7.** The method of one of the preceding claims, wherein the valve model (2, 3) comprises a plurality of commissure points (4) positioned along the circumference of the closed curve (9), wherein each commissure point (4) defines a starting point of a commissure line (9) between two leaflets (6) of the heart valve (52), and wherein a 2D section of the at least one 2D section is partly delineated by at least one commissure line (5) and corresponds to a leaflet.

**8.** The method of any one of the preceding claims, wherein a 2D section of the at least one 2D section (16) within the closed curve (9) corresponds to a physiological feature, wherein preferably the physiological feature is a flow phenomenon (7).

**9.** The method of any one of the preceding claims, wherein the valve model (2, 3) is or is based on a 3D valve model (2) of the annulus which is positioned and oriented within the 3D or 4D volume data set (32) at the position and orientation of the annulus of the heart valve (52),
and wherein the projection direction (36) extends at least approximately perpendicular to a plane fitted to the 3D valve model (2) of the annulus.

**10.** The method of any one of the preceding claims, wherein the valve model (2, 3) is based on or is correlated to a topology-based 2D model (3) of the heart valve.

**11.** The method of any one of the preceding claims, comprising a further step of receiving user input data for creating or adjusting at least a 2D section (16) within the valve model (2,3) via a user interface (10), wherein in particular the topology-based 2D model (3) is displayed to the user, and any adjustment of a 2D section (16) made on the topology-based 2D model (3) by the user are transferred to the valve model (2).

**12.** The method of any one of the preceding claims, wherein the visualisation is a dynamic 4D visualisation of the heart valve (52), and wherein the projection direction (36) of the topology-based 2D (3) model is adjusted according to the movement of the heart valve (52).

**13.** The method of any one of the preceding claims, wherein the volume-of-interest (42) extends into the 3D or 4D volume data set (32) up to a set height along the projection direction (36), preferably a height which is proportional to a dimension of the topology-based 2D (3) model.

**13.** Computer program including program code that, when executed on a control unit (16), is configured to execute the method according to any one of the preceding claims.

**15.** A system for analysing a heart valve (52) including a user interface configured to visualise a 3D or 4D volume data set (32), and a control unit configured to execute the method according to any one of claims 1 to 13.

## Amended claims

### Amended claims in accordance with Rule 137(2) EPC.

1. A computer-implemented method for analysing a heart valve (52) of a subject, the method comprising the steps of:
(a) providing a 3D or 4D volume data set (32) of a heart valve (52) of a subject;
(b) providing a valve model (2, 3) of the heart valve (52), wherein the valve model (2, 3) is adjusted to the morphology of the heart valve (52), and
wherein the valve model (2, 3) comprises a closed curve (9, 12) representing the annulus of the valve, wherein the area within the closed curve (9, 12) comprises at least one 2D section (16), each 2D section (16) corresponding to a morphological or physiological feature of the heart valve (52); and
(c) projecting the valve model (2, 3) into the 3D or 4D volume data set (32) along a projection direction (36) to define a volume-of-interest (42), wherein the volume-of-interest (42) includes at least one 3D region (26), each 3D region (26) being defined by the projection of a corresponding 2D section of the at least one 2D section (16);
(d) labelling each of the voxels in the at least one 3D region (26) with a label indicative of the morphological or physiological feature of the corresponding 2D section (16).

2. The method of claim 1, comprising a further step of
(e) providing a visualisation of the 3D or 4D volume data set (32) of the heart valve (52), the visualization comprising information from the projected valve model (2, 3), in particular wherein voxels having different labels are visualized differently.

3. The method of claim 2, wherein voxels in the at least one 3D region (26) of the volume-of-interest (42) are represented in a colour depending on their label.

4. The method of claim 2 or 3, wherein the visualisation includes visualizing a 2D cut plane (44) through the 3D or 4D volume data set (32).

5. The method of any one of claim 2 to 4, wherein the voxels within a 3D region of the at least one 3D region (26) are excluded from the visualisation and/or are represented in a neutral colour.

6. The method of any one of claims 2 to 5, wherein voxels which are outside of the volume-of-interest (42) are not visualized.

7. The method of one of the preceding claims, wherein the valve model (2, 3) comprises a plurality of commissure points (4) positioned along the circumference of the closed curve (9), wherein each commissure point (4) defines a starting point of a commissure line (9) between two leaflets (6) of the heart valve (52), and wherein a 2D section of the at least one 2D section is partly delineated by at least one commissure line (5) and corresponds to a leaflet.

8. The method of any one of the preceding claims, wherein a 2D section of the at least one 2D section (16) within the closed curve (9) corresponds to a physiological feature, wherein preferably the physiological feature is a flow phenomenon (7).

9. The method of any one of the preceding claims, wherein the valve model (2, 3) is or is based on a 3D valve model (2) of the annulus which is positioned and oriented within the 3D or 4D volume data set (32) at the position and orientation of the annulus of the heart valve (52),
and wherein the projection direction (36) extends at least approximately perpendicular to a plane fitted to the 3D valve model (2) of the annulus.

10. The method of any one of the preceding claims, wherein the valve model (2, 3) is based on or is correlated to a topology-based 2D model (3) of the heart valve.

11. The method of any one of the preceding claims, comprising a further step of receiving user input data for creating or adjusting at least a 2D section (16) within the valve model (2,3) via a user interface (10), wherein in particular the topology-based 2D model (3) is displayed to the user, and any adjustment of a 2D section (16) made on the topology-based 2D model (3) by the user are transferred to the valve model (2).

12. The method of any one of the preceding claims, wherein the visualisation is a dynamic 4D visualisation of the heart valve (52), and wherein the projection direction (36) of the topology-based 2D (3) model is adjusted according to the movement of the heart valve (52).

13. The method of any one of the preceding claims, wherein the volume-of-interest (42) extends into the 3D or 4D volume data set (32) up to a set height along the projection direction (36), preferably a height which is proportional to a dimension of the topology-based 2D (3) model.

14. Computer program including program code that, when executed on a control unit (16), is configured to execute the method according to any one of the preceding claims.

15. A system for analysing a heart valve (52) including a user interface configured to visualise a 3D or 4D volume data set (32), and a control unit configured to execute the method according to any one of claims 1 to 13.
